# EUROPEAN PATENT SPECIFICATION

(11) **EP 0 697 400 B1**
(45) Date of publication and mention of the grant of the patent: **08.04.1998**
(21) Application number: 95112938.6
(22) Date of filing: 17.08.1995
(51) Int. Cl.: C07C 235/06, C08F 20/36, C08F 20/68, C08F 16/26, C08F 112/14, C08F 212/14, C08F 120/36, C08F 120/68, C08F 220/36, C08F 220/68

(54) **Amide monomer and polymer prepared therefrom**
Amidmonomer und dessen Polymer
Monomère à base d'amide et polymère préparé à partir de ces derniers

(30) Priority: 19.08.1994 JP 195433/94
(43) Date of publication of application: 21.02.1996
(73) Proprietor: Nippon Paint Co., Ltd., Osaka-shi Osaka-fu (JP)
(72) Inventor: Adachi, Koji, Suita-shi, Osaka-fu (JP); Tsuboniwa, Noriyuki, Higashiosaka-shi, Osaka-fu (JP); Urano, Satoshi, Tsuzuki-gun, Kyoto-fu (JP)
(74) Representative: Hansen, Bernd, Dr. Dipl.-Chem.

(56) References cited:
- EP-A- 0 374 866
- EP-A- 0 437 728
- EP-A- 0 489 930
- US-A- 2 458 420
- DATABASE WPI Week 9440 Derwent Publications Ltd., London, GB; AN 94-322147 XP002000611 & JP-A-06 247 917 (NIPPON SHOKUBAI CO LTD) , 6 September 1994
- JOURNAL OF THE AMERICAN OIL CHEMISTS' SOCIETY, vol. 43, no. 9, 1966, CHAMPAIGN US, pages 542-545, XP000195938 JANE S. NELSON ET AL.: "Castor-Based Derivatives: Synthesis of Some Acrylate Esters"

## Description

The present invention relates to a novel monomer and a polymer prepared therefrom, which are suitable for preparation of a binder for paint.

### BACKGROUND OF THE INVENTION

JP-A-06 247 917 relates to the production of N-substituted-carbamoyl ethyl (meth)acrylates; US-A-2 458 420 relates to acrylic esters-amides and polymers thereof.

Amide group is a polar group having an ability of forming hydrogen bond and has high hydrophilic nature. Accordingly, an amide monomer having a primary amide group therein is widely used for modification of polymer material for paint, paper and fiber.

Among such amide monomers, a (meth)acrylamide is most popular and easily obtainable. The (meth)acrylamide gives a polymer having a very high glass transition temperature of 160 to 250 °C and having high hydrophilic nature and poor solubility in organic solvent. However, if the (meth)acrylamide is used in a high monomer content and applied to paint, it adversely affects on film forming ability and water resistance of cured film.

### SUMMARY OF THE INVENTION

The present invention provides a novel amide monomer and a polymer prepared therefrom, which has suitable glass transition temperature (Tg) and solvent solubility as well as provides paint having excellent firm forming properties and water resistance. The novel amide monomer is represented by 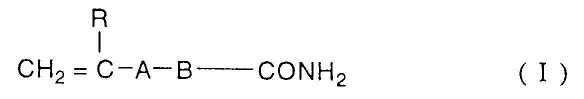 wherein R represents a hydrogen atom or an alkyl group having 1 to 4 carbon atoms and A represents a bonding portion indicated by 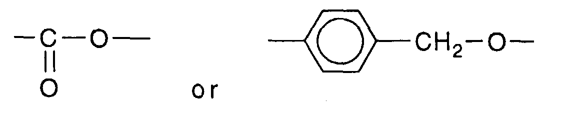 B represents a bonding portion shown by 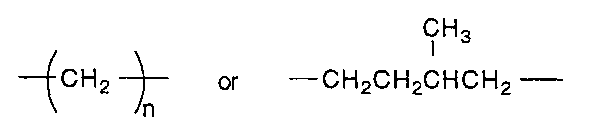 in which n represents an integer of 3 to 10.

The present invention also provide a polymer prepared from the above amide monomer.

### DETAILED DESCRIPTION OF THE INVENTION

One of the embodiment of the present invention is to provide an amide monomer represented by 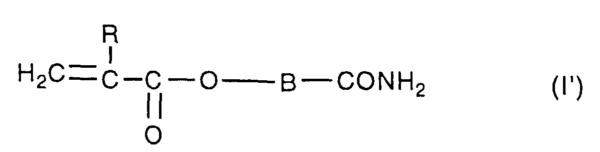 (wherein R represents a hydrogen atom or an alkyl group having one to four carbon atoms, preferably a methyl group, and B is the same as mentioned above.)
The amide monomer (I') may be obtained by reacting a compound represented by 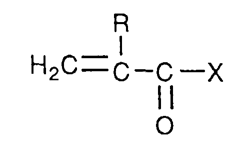 (wherein X represents a chlorine atoms, a hydroxyl group or an alkoxy group having upto 8 carbon atoms, and R is the same as mentioned above),
with a compound represented by

J―B―CONH₂

(wherein J represents a chlorine atom or a hydroxyl group, and B is the same as mentioned above.)
In the reaction, the combination of X and J is as follow:
X is a chlorine atom, J is a hydroxyl group;
X is a hydroxyl group, J is a chlorine atom or a hydroxyl group; and
X is an alkoxy group, J is a hydroxyl group.

For example, if the amide monomer is obtained by the reaction represented by 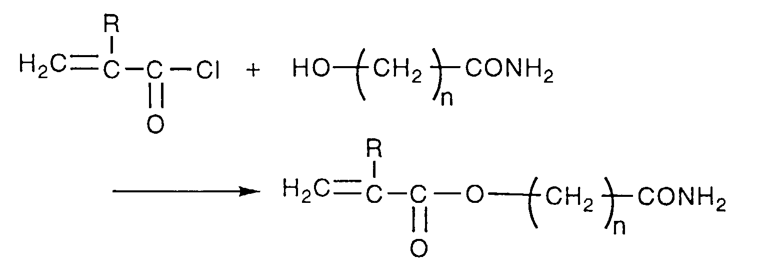 the reaction may be conducted at a temperature of 0 to 100 °C, preferably 0 to 40 °C in a solvent in the presence of a basic catalyst, followed by isolating the amide monomer by art-known methods, for example, distillation, recrystallization or chromatography.

The basic catalyst employed therein includes an aliphatic tertiary amine, such as triethylamine; an aromatic amine, such as pyridine; an inorganic base, such as sodium carbonate and potassium carbonate. The solvent used in the reaction is one which has no active hydrogen, for example hydrocarbon, halogenated hydrocarbon, ketone, ether, ester and the like. Representative examples of the solvent is an aliphatic hydrocarbon, such as pentane, hexane and heptane; an aromatic hydrocarbon, such as benzene, toluene and xylene; an alicyclic hydrocarbon, such as cyclohexane, methyl cyclohexane and decalin; a petroleum hydrocarbon, such as petroleum ether and petroleum benzine; a halogenated hydrocarbon, such as chloroform and 1,2-dichloroethane; an ether, such as ethyl ether, isopropyl ether, anisole, dioxane and tetrahydrofrane; a ketone, such as acetone, methyl ethyl ketone, methyl isobutyl ketone, chclohexanone, acetophenone and isophorone; an ester, such as ethyl acetate and butyl acetate; acetonitrile; dimethylformamide; N-methylpyrrolidone; dimethylsulfoxide; and a mixture thereof.

The compound represented by 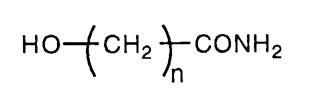 is known to the art, for example Journal of American Chemical Society, Vol. 84, page 365, 1962 wherein a compound with n=3 is disclosed, Journal of American Chemical Society, Vol. 73, pages 4794 and 4798, 1951 wherein a compound with n=4 is disclosed, and Journal of Japanese Chemical Association, pages 1013 and 1015, 1977 wherein a compound with n=5 is disclosed. Compounds with n=6 and 7 are also known to the art.

When the amide monomer is obtained by the following chemical reaction: 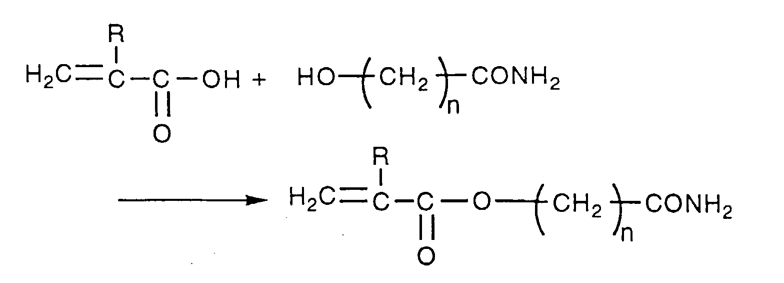 the reaction may be conducted at a temperature of 50 to 200 °C, preferably 80 to 120 °C in the presence of acid catalyst in a solvent by dehydration. The reaction may be conducted at a reduced pressure to remove water out of the reaction system.

Examples of the acid catalyst are an organic protonic acid, such as p-toluenesulfonic acid; an inorganic protonic acid, such as hydrochloric acid and sulfuric acid; a Lewis acid, such as trifluoro borate. The solvent is one which has no active hydrogen, as mentioned above. If necessary, a dehydrating agent may be added to the reaction system. Preferred dehydrating agents are a carbodiimide compound, such as dicyclohexylcarbodiimide; a phosphorus compound such as phosphorus oxychloride, phosphorus trichloride and phosphorus tetrachloride; and molecular sieves.

The amide compound (I') may also obtained by the following reaction: 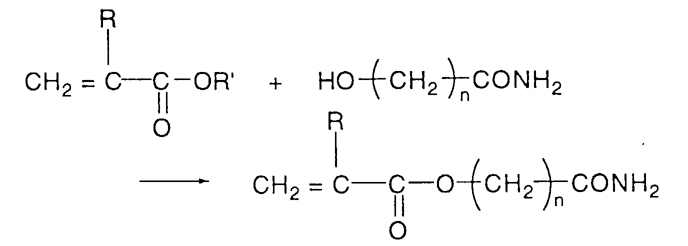 (wherein R' represents a lower alkyl, preferably an alkyl group having 1 to 8 carbon atoms, and R and n is the same as mentioned above.)
The reaction is conducted at a temperature of 50 to 200 °C, preferably 80 to 150 °C in the presence of a basic or acidic catalyst in a solvent.

The basic catalyst includes an alkali metal alcoholate, such as sodium methylate and potassium t-butylate; an alkali metal hydroxide, such as sodium hydroxide and potassium hydroxide. The acidic catslyst includes a protonic acid, such as p-toluenesulfonic acid and sulfuric acid; a Lewis acid, such as a titanium alkoxide (e.g. titanium tetraethoxide and titanium tetraisopropoxide) and a tin compound (e.g. dibutyltin oxide, dibutyltin dilaurate and dibutyltin octoate). The solvent can be one which has no active hydrogen, as mentioned above.

The other compound of the amide monomer (I) of the preset invention is represented by 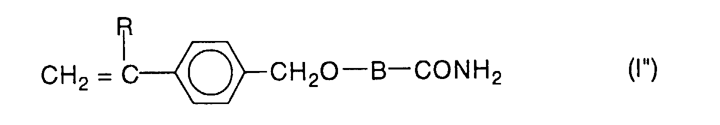 (wherein R represents a hydrogen atom or an alkyl group having 1 to 4 carbon atoms, preferably a hydrogen atom, B is the same as mentioned above.)
The compound (I") may be prepared by reacting a compound represented by 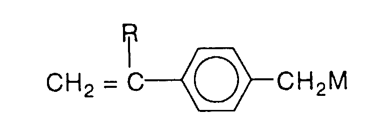 (wherein R is the same as mentioned above, M is a chlorine atom or a bromine atom),
with a compound represented by

HO―B―CONH₂

(wherein B is the same as mentioned above.)

The reaction can be conducted at a temperature of 0 to 200 °C, preferably 20 to 120 °C in the presence of basic catalyst in a solvent. The basic catalyst employed includes sodium hydroxide, potassium hydroxide, sodium carbonate, calcium carbonate, potassium t-butylate, and sodium methylate. The solvent for the reaction is one which has no active hydrogen, as mentioned above.

The present invention further provides a polymer prepared from the monomer (I). The polymer can be a homopolymer of the monomer (I) or a copolymer of the monomer (I) with other monomers copolymerizable therewith.

The homopolymer of the monomer (I) may be represented by the following formula: 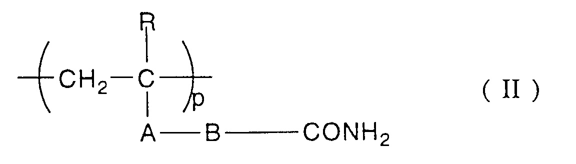 (wherein R, A and B are the same as mentioned above, p is an integer.)

The other copolymerizable monomers are ethylenic unsaturated monomers having no reactivity with primary amide group, which are known to the art. The other copolymerizable monomers may be presented by the following formula: 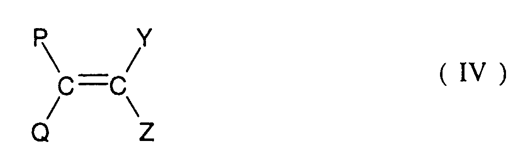 (wherein P, Q, Y and Z independently show a hydrogen atom; an alkyl group or halogenated alkyl having 1 to 6 carbon atoms, such as methyl, ethyl, propyl and chloromethyl; an alkenyl group having 1 to 4 atoms, such as allyl, butenyl; an aryl or halogenated aryl group having not more than 10 carbon atoms, such as phenyl, tolyl, ethylphenyl and chlorophenyl; an aralkyl or halogenated aralkyl having not more than 10 carbon atoms, such as benzyl or chlorobenzyl; a cyano group; a mono- or di-alkylamino group having 1 to 6 carbon atoms, such as amino, methylamino and dimethylamino; a cyanaminoalkyl group having 1 to 6 carbon atoms, such as cynaminomethyl and cyanaminoethyl; an alkylcarbonyloxy group having 1 to 6 carbon atoms, such as methylcarbonyloxy and ethylcarbonyloxy; an arylcarbonyloxy group having not more than 10 carbon atoms, such as phenylcarbonyloxy; a (Ph)ₖ-(CH₂)ₗ-CORₐ group (wherein Ph is a phenylene group, Rₐ is hydroxyl, an alkoxy group having 1 to 10 carbon atoms, an aryloxy group having not more than 10 carbon atoms, an aralkyloxy group having not more than 10 carbon atoms, a hydroxyalkoxy group having 1 to 6 carbon atoms, amino or a mono- or di-alkylamino group having 1 to 6 carbon atoms, k is 0 or 1, l is an integer of 0 to 6), such as -COOH,COOCH₃, -CH₂-COOCH₃, -COOCH₂CH₂OH and -CONH₂; or a(Ph)ₒ-(CH₂)ₚ-SO₂R_{b} (wherein Ph is the same as mentioned above, R_{b} is a hydroxyl group, an alkoxy group having 1 to 10 carbon atoms or an aralkyloxy group having not more than 10 carbon atoms, o is 0 or 1, p is an integer of 0 to 6), such as -(Ph)-SO₃H.)

Typical examples of the other monomers (IV) are a mono-olefin or diolefin, such as styrene, alpha-methylstyrene, alpha-ethylstyrene, 2-methyl-1-butene, ethylene, propylene, butene, amylene, hexene, 1,3-butadiene, and isoprene; a halogenated monoolefin or diolefin, such as alpha-chlorostyrene and alpha-bromostyrene; an organic or inorganic ester, such as vinyl acetate, vinyl propionate, vinyl butylate, vinyl benzoate, methyl methacrylate, ethyl methacrylate, propyl methacrylate, hexyl methacrylate, methyl acrylate, ethyl acrylate, propyl acrylate, allyl chloride, allyl cyanamide, allyl acetate, allyl propionate, allyl butylate, dimethyl maleate, diethyl maleate, dimethyl maleate, diethyl fumarate, dimethacryl fumarate and diethyl glutaconate; an organic nitrile, such as acrylonitrile, methacrylonitrile, ethacrylonitrile, 3-octene nitrile, croton nitrile and oleonitrile; an unsaturated acid, such as acrylic acid, methacrylic acid and crotonic acid; an unsaturated alcohol, such as a monoester of the above mentioned unsaturated acid with a glycol (e.g. ethylene glycol and propylene glycol), an adduct of 2-hydroxyethyl methacrylate with 1 to 5 mol of epsilon-caprolactone and the like; and an unsaturated sulfonic acid or a salt thereof, such as 2-sulfoethyl acrylate, and p-vinylbenzenesulfonic acid; and a mixture thereof.

The resulting homopolymer or copolymer may be represented by the following two repeating units 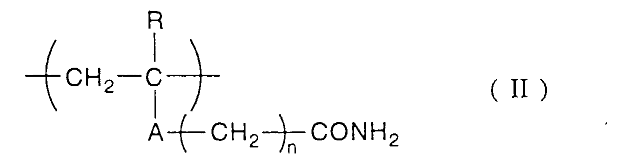 (wherein R, A and n are the same as mentioned above) 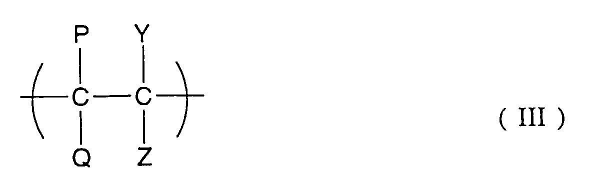 (wherein P, Q, Y and Z are the same as mentioned above.) If the number of the unit (II) is expressed "s" and the number of the unit (III) is expressed "t", the number "s" is a positive integer and the number "t" is 0 or a positive integer. If the number "t" is 0, the resulting polymer is a homopolymer represented by only the unit (II). The polymer of the present invention preferably has an amide group in the unit (II) in an amount of 0.1 to 28 % by weight and a number average molecular weight of 500 to 100,000. The number average molecular weight is determined by GPC (Gel Permeation Chromatography).

The polymer of the present invention may be prepared by polymerizing the monomer (I) and optionally the monomer (IV)in a solvent without active hydrogen, alcohols or aqueous medium. For example, a reaction vessel is charged with the solvent and optionally a portion of the monomers and reacted at a temperature of 20 to 200 °C, preferably 80 to 150 °C. Into the reaction vessel, a remaining monomer solution and a polymerization initiator are added dropwise over 0.5 to 5 hours, preferably 1.5 to 3 hours. After completion of the dropping, the temperature maintains for 0.5 to 2 hours to form a solution containing the polymer of the present invention.

The polymerization initiator used for the polymerization is known to the art and is not limited, but for example an organic peroxide, such as benzoyl peroxide, t-butyl perbenzoate, t-butyl peroxide, hydroperoxide, cummen hydroperoxide, di-t-butyl peroxide and butyl peroctoate; an azo compound, such as 2,2'-azobisisobutylonitrile, dimethyl azodiisobutylate, 2,2'-azobis(2,4-dimethylvaleronitrile; and a mixture thereof. The initiator may be present in an amount of 0.1 to 10 % by weight, preferably 0.5 to 5 % by weight based on the total amount of the monomers.

In the reaction, a chain transfer agent may be added in order to control molecular weight. The chain transfer agent is known to the art and includes alpha-methyl styrene dimer, octylmercaptane, dodecylmercaptane, thioglycolic acid and a mixture thereof. The agent may be added in an amount of 0.5 to 20 % by weight based on the total weight of the monomers.

The polymer thus obtained consists of a main chain of carbon and carbon bond and has an amide group in an amount of 0.1 to 28 % by weight, preferably 1 to 20 % by weight and a molecular weight of 500 to 100,000. The production of the polymer can be identified by a strong absorption at near 1670 cm⁻¹ in IR spectrum and the coincidence of actual nonvolatile content in the resulting polymer with a calculated nonvolatile content.

The polymer solution can used neatly, but may be diluted with the solvent mentioned above. If necessary, the polymer solution may be condensed to a suitable concentration.

### EXAMPLES

The present invention is illustrated by the following Examples which, however, are not to be construed as limiting the present invention to their details.

### Example 1

### Synthesis of 4-methacryloyloxy butyloamide

A 200 ml three-necked reaction vessel, equipped with a stirrer, a thermometer, a reflux condenser, a dropping funnel and an oil bath, was charged with 7.0 g (0.07 mol) of gamma-hydroxybutyloamide, 6.9 g (0.07 mol) of triethylamine, 50 g of 1,4-dioxane and 70 mg of a polymerization inhibitor, and mixed in nitrogen atmosphere. Then, 10 g of a 1,4-dioxane solution of 7.1 g (0.07 mol) of methacrylic chloride was added over one hour at 25 °C, and then mixed for another 1.5 hour. The content exotermed to 33 °C. The precipitated hydrochloric acid salt of triethylamine was filtered out and the filtrate was condensed by an evaporator to obtain light yellow oily substance. The substance was separated by silica gel chromatography (a developer: methylene chloride - methylene chloride/methanol = 10/1) to obtain white rude crystal. The crystal was recrystallized from acetone to obtain 5.3 g of colorless needle crystal of 4-methacryloyloxy butyloamide (46 % yield).

The chemical formula: 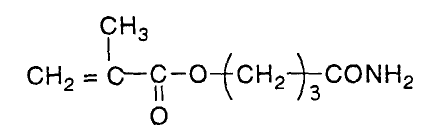
Melting point = 33 to 34 °C
NMR Spectrum: ¹H NMR (360MHz, CDCl₃, δ), 1.95 (s, 3H, CH₃), 2.05(quint, 2H, CH₂CH₂CH₂), 2.33 (t, 2H, CH₂, CH₂CON), 4.22 (t, 2H, COOCH₂), 5.58,6.11 (s, each 1H, CH₂=), 5.65 (bs, 1H, NH), 5.72(bs, 1H, NH)
IR(KBr plate, cm⁻¹): 3350, 3180 (N-H stretching), 1720(COO), 1670(CON), 1660(N-H deformation), 1640(CH₂=C)

### Example 2

### Synthesis of 6-methacryloyloxy caproamide

A 500 ml three-necked reaction vessel, equipped with a stirrer, a thermometer, a reflux condenser, a dropping funnel and an oil bath, was charged with 20.0 g (0.15 mol) of 6-hydroxycaproamide, 17.0 g (0.17 mol) of triethylamine, 50 g of acetone and 300 mg of a polymerization inhibitor, and mixed in nitrogen atmosphere. Then, 30 g of an acetone solution of 17.6 g (0.17 mol) of methacrylic chloride was added over one hour at a content temperature of 20 °C, and then mixed for another 1 hour. The content exotermed to 36 °C. The reaction mixture was concentrated by an evaporator, to which ethyl acetate was added to filter off hydrochloric acid salt of triethylamine and the filtrate was condensed by an evaporator to obtain light yellow oily substance. The substance was separated by silica gel column chromatography (a developer: methylene chloride → methylene chloride/methanol = 10/1) to obtain white rude crystal. The crystal was recrystallized from toluene to obtain 17.1 g of colorless needle crystal of 6-methacryloyloxy caproamide (56 % yield).

The chemical formula: 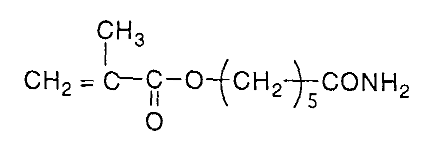
Melting point = 40 to 41 °C
NMR Spectrum: ¹H NMR (360MHz, CDCl₃, δ), 1.45(quint, 2H,-(CH₂)₂CH₂(CH₂)2-), 1.65-1.80(m, 4H, -CH₂CH₂CH₂CH₂CH₂-), 1.94 (s, 3H, CH₃), 2.25 (t, 2H, CH₂CON), 4.15 (t, 2H, COOCH₂), 5.55, 6.09 (s, each 1H, CH₂=), 5.6-5.8 (bs, 2H, NH₂)
IR(KBr plate, cm⁻¹): 3360, 3200 (N-H stretching), 1710 (COO), 1660 (CONH₂), 1630 (CH₂=C)

### Example 3

### Synthesis of 6-(4-vinylbenzyl) caproamide

A 100 ml three-necked reaction vessel, equipped with a stirrer, a thermometer, a reflux condenser, a dropping funnel and an oil bath, was charged with 5.0 g (0.04 mol) of 6-hydroxycaproamide, 1.5 g (0.04 mol) of NaOH, 50 g of 1,4-dioxane and 200 mg of a polymerization inhibitor, and mixed in nitrogen atmosphere. Then, 10 g of a 1,4-dioxane solution of 5.8 g (0.04 mol) of chloromethylstyrene was added over one hour at a content temperature of 50 °C, and then mixed for another 0.5 hour. It was then heated to 100 °C, to which 0.6 g (0.004 mol) of potassium iodide was added and mixed for 6 hours with heating. The precipitated hydrochloric acid salt was filtered out and the filtrate was condensed by an evaporator to obtain orange semisolid substance. The substance was separated by reverse phase (C18) chromatography (a developer: methanol/water = 1/1) to obtain white rude crystal. The crystal was recrystallized from acetone to obtain 1.7 g of colorless needle crystal of 6-(4-vinylbenzyl) caproamide (18 % yield).

The chemical formula: 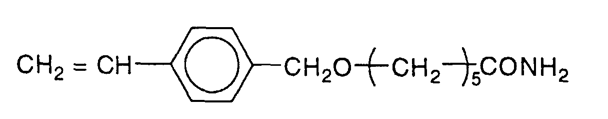
Melting point = 105 to 107 °C
NMR Spectrum: ¹H NMR (360MHz, CDCl₃, δ), 1.35-1.50(m, 2H,-(CH₂)₂CH₂(CH₂)2-), 1.60-1.70(m, 4H, -CH₂CH₂CH₂CH₂CH₂-), 2.22(t, J=7.4 Hz, 2H, CH₂CON), 3.47(t, J=6.4 Hz, 2H, -OCH₂CH₂-), 4.48(s, 2H, ArOCH₂-), 5.24(dd, Jab=0.9 Hz, Jbc=10.9 Hz, 1H, Ha(trans)Hb(cis)C=CHc-), 5.57(dd, Jab=0.9 Hz, Jac=1.76 Hz, Ha(trans)Hb(cis)C=CHc-), 5.45(bs, 1H, NH), 5.55(bs, 1H, NH), 6.71 (dd, Jac=17.6 Hz, Jac=10.9 Hz, 1H, Ha(trans)Hb(cis)C=CHc-), 7.29(d, J=8, 2 Hz, 2H, aromatic), 7.39(d, J=8.2 Hz, 2H, aromatic)
IR(KBr plate, cm⁻¹): 3350, 3180 (N-H stretching), 1670(CONH₂), 1635(C=C), 1090(C-O-C)

### Example 4

### Synthesis of polymer of 4-methacryloyl butyloamide

A 200 ml three-necked reaction vessel, equipped with a stirrer, a thermometer, a reflux condenser, a dropping funnel and an oil bath, was charged with 15.0 g of butyl acetate and heated to 105 °C. The dropping funnel was charged with 5.0 g of styrene, 5.0 g of methyl methacrylate, 5.0 g of 2-ethylhexyl methacrylate, 5.0 g of 4-methacryloyloxy butyloamide, 0.16 g of t-butylperoxy(2-ethyl)hexanoate and 30.0 g of butyl acetate, and added dropwise into the reaction vessel at a content temperature of 105 to 110 °C for 3 hours in nitrogen atmosphere with mixing. After completion of the addition, it was mixed for 20 minutes, and then a solution of 0.2 g of t-butylperoxy(2-ethyl)hexanoate and 2.0 g of butyl acetate was added dropwise over 15 minutes. Mixing continued for another 1.5 hour with keeping the temperature at 105 °C. The mixture was cooled to room temperature to obtain a polymer solution. It was identified by IR spectrum (KBr plate) showing a particular absorption of carbonyl group at 1670 ⁻¹. It had a nonvolatile content of 29.1 % (theoretical value = 29.8 %), 7 % by weight of amide group content and a number average molecular weight of 8,900.

### Example 5-7

Polymers were prepared as generally described in Example 4 with the exception that the ingredients shown in the following Table 1 were employed. The physical properties of the resulting polymers are shown in Table 2.

**Table 1**

| Ingredients | Example 5 (g) | Example 6 (g) | Example 7 (g) |
|---|---|---|---|
| Styrene | 5.0 | 5.0 | 5.0 |
| Methyl methacrylate | 5.0 | 5.0 | 5.0 |
| 2-Ethylhexyl methacrylate | 5.0 | 5.0 | 5.0 |
| 6-Methacryloyloxy caproamide | 5.0 | 5.0 | - |
| 6-(4-vinylbenzyl)caproamide | - | - | 5.0 |
| Solvent | Butyl acetate 45.0 | Toluene 50.0 | Butyl acetate 45.0 |

### Example 8

A 50 ml three necked flask was charged with 4 g of dimethylformamide (DMF) and purged with nitrogen gas, followed by heating to 70 °C. A dropping funnel was charged with 3 g of 4-methacryloyloxy butyloamide, 0.06 g of 2,2'-azobis(2,4-dimethylvaleronitrile) and 10 g of DMF and added dropwise into the reaction flask over 2 hours. After completion of the addition, it was mixed at 70 °C for 4 hours and a solution of 0.3 g of 2,2'-azobis(2,4-dimethylvaleronitrile) and 1 g of DMF was added dropwise for 10 minutes, followed by mixing for 50 minutes with heating. After cooling it, the reaction mixture was added to toluene to precipitate a homopolymer of 1.8 g (60 %). It was filtered and dried, and then subjected to IR spectrum and glass transition temperature. Other physical properties are shown in Table 2.
IR (cm⁻¹): 3320, 3180 (NH), 1710 (COO), 1660 (CONH₂)
Tg 36 °C (determined by a thermal analysis apparatus available from Seiko Electronic Ind., Co. as SSC-5200 at a heating rate of 10 °C/min.)

### Example 9

A flask was charged with 100 g of methyl isobutyl ketone and heated to 105 °C, into which a mixture of 10 g of styrene, 10 g of methyl methacrylate, 14 g of 2-ethylhexyl methacrylate, 6 g of methacrylic acid, 60 g of 4-methacryloyloxy butyloamide, 150 g of methyl isobutyl ketone and 1 g of t-butylperoxy(2-ethyl)hexanoate was added dropwise for 3 hours. After completion of the addition, it was mixed for 20 minutes, to which a solution of 10 g of methyl isobutyl ketone and 1.3 g of t-butylperoxy(2-ethyl)hexanoate was added dropwise for 15 minutes. It was mixed for 1.5 hour and cooled to obtain a polymer solution. The physical properties of the resulting polymer are shown in Table 2.

### Example 10

A flask was charged with 100 g of butyl acetate and heated to 105 °C, into which a mixture of 5 g of styrene, 4 g of 2-ethylhexyl methacrylate, 10 g of 2-hydroxyethyl acrylate, 6 g of methacrylic acid, 60 g of 6-methacryloyloxy butyloamide, 60 g of butyl acetate and 1 g of t-butylperoxy(2-ethyl)hexanoate was added dropwise for 3 hours. After completion of the addition, it was mixed for 20 minutes, to which a solution of 10 g of butyl acetate and 1.3 g of t-butylperoxy(2-ethyl)hexanoate was added dropwise for 15 minutes. It was mixed for 1.5 hour and cooled to obtain a polymer solution. The physical properties of the resulting polymer are shown in Table 2.

**Table 2**

| Ex. No. | Theoretical nonvolatile content (%) | Measured nonvolatile content (%) | Amide group content (% by weight) | Mn | IR(υ_{c=o},cm⁻¹) |
|---|---|---|---|---|---|
| 4 | 29.8 | 29.1 | 7 | 8900 | 1670 |
| 5 | 30.8 | 29.8 | 7 | 9000 | 1670 |
| 6 | 28.6 | 28.0 | 7 | 8800 | 1670 |
| 7 | 30.8 | 30.1 | 4.45 | 8700 | 1670 |
| 8 | - | - | 28.0 | 12000 | 1660 |
| 9 | 27.8 | 27.2 | 16.8 | 9200 | 1660 |
| 10 | 30.0 | 29.6 | 19.8 | 7900 | 1660 |

### Example 11

Evaluation of solubility of amide monomer

Each 0.1 g of the amide monomer obtained in Examples 1-3 was charged in 1 g of solvent and its solubility was evaluated by eyes. Good shows completely solved. Fairly good shows a portion of the monomer remaining but if heated to 40 °C, completely solved. Poor shows a portion of the monomer remaining even if heated to 40 °C.

**Table 3**

| Example No. | Amide monomer | Solvent | |
|---|---|---|---|
| | | Butyl acetate | Toluene |
| - | Acrylamide | Fairly good | Poor |
| - | Methacrylamide | Fairly good | Poor |
| - | Methacryloyloxy acetoamide | Fairly good | Poor |
| 1 | 4-Methacryloyloxy butyloamide | Good | Fairly good |
| 2 | 6-Methacryloyloxy caproamide | Good | Good |
| 3 | 6-(4-vinylbenzyl) caproamide | Good | Good |

As clearly understood from the above results, the amide monomer of the present invention has very good solvent solubility in comparison with the art-known amide monomers.

### Example 12

### Synthesis of 5-methacryloyloxy-3-methylvaleroamide

A 500 ml three-necked reaction vessel, equipped with a stirrer, a thermometer, a reflux condenser, a dropping funnel and a cooling bath, was charged with 52.4 g (0.4 mol) of gamma-hydroxy-beta-methylvaleroamide, 41.8 g (0.4 mol) of methacrylic chloride, 400 g of 1,2-dichloroethane and 500 mg of a polymerization inhibitor, and mixed in nitrogen atmosphere. Then, 50 ml of a 1,2-dichloroethane solution of 40.4 g (0.4 mol) of triethylamine was added over two hours at 25 °C, and then mixed for another 1.5 hour. Since the content exotermed during the addition, the reaction vessel was cooled by ice to keep the temperature constant. The precipitated hydrochloric acid salt of triethylamine was filtered out and the filtrate was condensed by an evaporator to obtain light yellow oily substance. The substance was separated by silica gel column chromatography (a developer: hexane/ethyl acetate= 3/1 → 1/4) to obtain light yellow oily material of 5-methacryloyloxy-3-methylvaleroamide (10.3 g; 13 % yield)

The chemical formula: 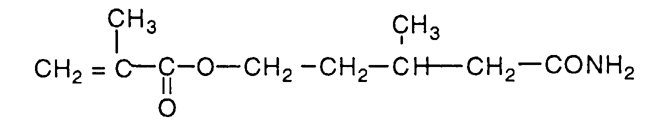
Viscosity 65 cp (EM viscometer available from Tokyo Keiki Co. at 30 °C)
NMR Spectrum: ¹H NMR (360MHz, CDCl₃, δ), 1.024 (d, 3H, CH₃), 1.587, 1781 (each m, each 1H, CH₂), 1.938 (s, 3H, CH₂=C(CH₃CO), 2.038, 2.096 (each m, each 1H, CH₂), 2.278 (m, 1H, CH), 4.191 (m, 2H, OCH₂), 5.561, 6.097 (each s, each 1H, CH₂=C), 5.86, 6.10 (each brs, each 1H, NH₂)
¹³C-NMR (360MHz, CDCl₃, δ), 18.203 (CH₂=C(CH₃)CO), 19.533 (CH₃), 27.829, 35.115 (each CH₂), 43.074 (CH), 62.651 (OCH₂), 125.448, 136.232 (CH₂=C(CH₃)CO), 167.493 (COO), 174.793 (CONH₃)
IR(KBr film cm⁻¹): 3429, 3352, 3200 (N-H stretching), 2961, 2932, 2876 (CH stretching), 1716(COO), 1666(CONH), 1637 (C=C)

### Example 13

### Synthesis of 5-methacryloyloxy valeroamide

A 500 ml three-necked reaction vessel, equipped with a stirrer, a thermometer, a reflux condenser, a dropping funnel and a cooling bath, was charged with 46.8 g (0.4 mol) of gamma-hydroxyvaleroamide, 41.8 g (0.4 mol) of methacrylic chloride, 400 g of N,N-dimethylacetoamide and 500 mg of a polymerization inhibitor, and mixed in an ice bath in nitrogen atmosphere. Then, 50 ml of an N,N-dimethylacetoamide solution of 40.4 g (0.4 mol) of triethylamine was added over two hours at 0 °C, and then mixed for another 1.5 hour. The precipitated hydrochloric acid salt of triethylamine was filtered out and the filtrate was extracted with about 800 ml of hexane three times, and N,N-dimethylacetoamide was removed as hexane solution by decantation. Insoluble portion in hexane was light yellow oily substance. The substance was separated by silica gel column chromatography (a developer: hexane/ethyl acetate= 1/1 → 1/2) to obtain light yellow oily material of 5-methacryloyloxyvaleroamide (4.7 g; 6.3 % yield)

The chemical formula: 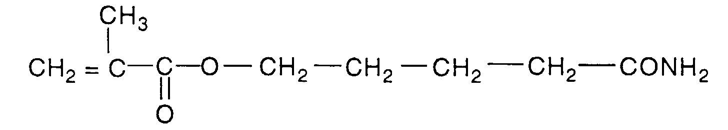
Viscosity 150 cp (EM viscometer available from Tokyo Keiki Co. at 25 °C)
NMR Spectrum: ¹H NMR (360MHz, CDCl₃, δ), 1.597-1.678 (m, 4H, CH₂), 1.861 (s, 3H, CH₂=C(CH₃)CO), 2.275-2.294 (m, 2H, CH₂CONH₂), 4.008-4.025 (m, 2H, OCH₂), 5.484, 6.020 (each s, each 1H, CH₂=C), 6.186, 6.1291 (each brs, each 1H, NH₂)
¹³C-NMR (360MHz, CDCl₃, δ), 17.994 (CH₂=C(CH₃)CO), 21.653, 27.831, 34.819 (each CH₂), 63.976 (OCH₂), 125.246, 136.027 (CH₂=C(CH₃)CO), 167.302 (COO), 175.648 (CONH₂)
IR(KBr film cm⁻¹): 3405, 3362, 3200 (N-H stretching), 2957, 2866 (CH stretching), 1718(COO), 1670(CONH), 1635 (C=C)

## Claims

1. An amide monomer represented by 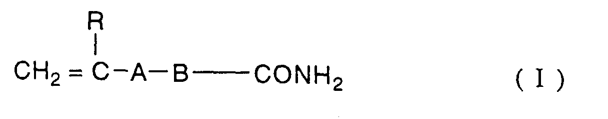 wherein R represents a hydrogen atom or an alkyl group having 1 to 4 carbon atoms and A represents a bonding portion indicated by 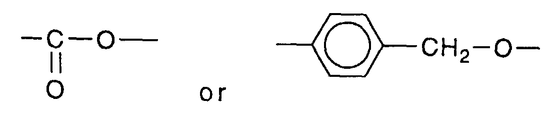 B represents a bonding portion shown by 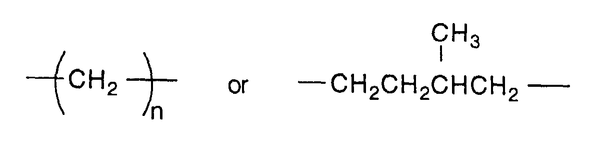 in which n represents an integer of 3 to 10.

2. The amide monomer according to claim 1 being represented by: 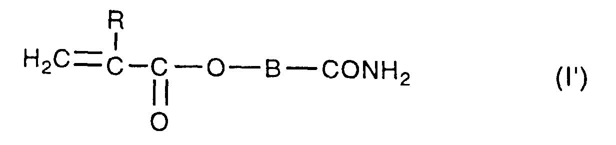 (wherein R and B are the same as mentioned above.)

3. The amide monomer according to claim 2 being obtained by reacting a compound represented by 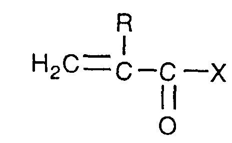 (wherein X represents a chlorine atoms, a hydroxyl group or an alkoxy group having upto 8 carbon atoms, and R is the same as mentioned above),
with a compound represented by
J―B―CONH₂
(wherein J represents a chlorine atom or a hydroxyl group, and B is the same as mentioned above.)

4. The amide monomer according to claim 3 wherein the combination of X and J is as follow:
X is a chlorine atom, J is a hydroxyl group;
X is a hydroxyl group, J is a chlorine atom or a hydroxyl group; and
X is an alkoxy group, J is a hydroxyl group.

5. The amide monomer according to claim 1 being represented by: 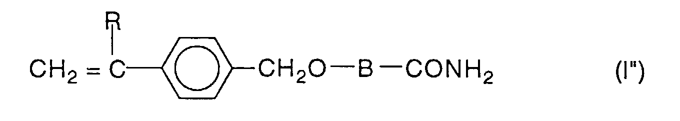 (wherein R and B are the same as mentioned above.)

6. The amide monomer according to claim 5 being prepared by reacting a compound represented by 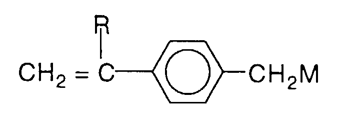 (wherein R is the same as mentioned above, M is a chlorine atom or a bromine atom),
with a compound represented by
HO―B―CONH₂
(wherein B is the same as mentioned above.)

7. A polymer prepared from the monomer according to claim 1.

8. A polymer according to claim 7 being represented by the repeating units: 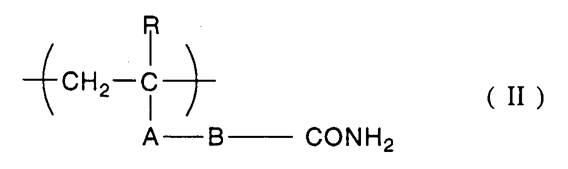 (wherein R, A and B are the same as mentioned above), 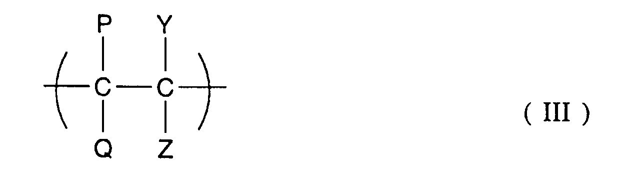 (wherein P, Q, Y and Z independently show a hydrogen atom; an alkyl group or halogenated alkyl having 1 to 6 carbon atoms; an alkene having 1 to 200 atoms; an aryl or halogenated aryl group having not more than 10 carbon atoms; an aralkyl or halogenated aralkyl having not more than 10 carbon atoms; a cyano group; a mono- or di-alkylamino group having 1 to 6 carbon atoms; a cyanaminoalkyl group having 1 to 6 carbon atoms; an alkylcarbonyloxy group having 1 to 6 carbon atoms; an arylcarbonyloxy group having not more than 10 carbon atoms; a (Ph)ₖ-(CH₂)ₗ-CORₐ group (wherein Ph is a phenylene group, Rₐ hydroxyl, an alkoxy group having 1 to 10 carbon atoms, an aryloxy group having not more than 10 carbon atoms, an aralkyloxy group having not more than 10 carbon atoms, a hydroxyalkoxy group having 1 to 6 carbon atoms, amino or a mono- or di-alkylamino group having 1 to 6 carbon atoms, k is 0 or 1, l is an integer of 0 to 6); or a -(Ph)ₒ-(CH₂)ₚ-SO₂R_{b} (wherein Ph is the same as mentioned above, R is a hydroxyl group, an alkoxy group having 1 to 10 carbon atoms or an aralkyloxy group having not more than 10 carbon atoms, o is 0 or 1, p is an integer of 0 to 6), wherein the number of the unit (II) is "s" (s is a positive integer) and the number of the unit (III) is "t" (t is 0 or a positive integer) and the amide group in the unit (II) is present in an amount of 0.1 to 28 % by weight and the polymer has a number average molecular weight of 500 to 100,000.

9. The polymer according to claim 8 being prepared by polymerizing the amide monomer (I) represented by: 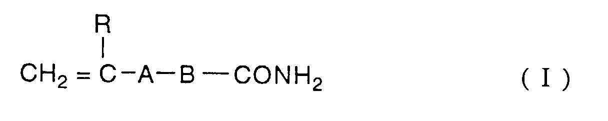 (wherein R, A and B are the same as mentioned above),
and optionally the monomer (IV) represented by: 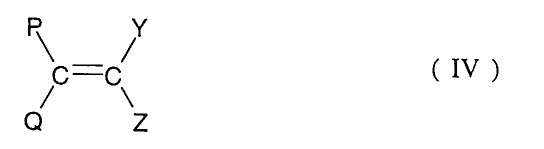 (wherein P, Q, Y and Z are the same as mentioned above.)

## Patentansprüche

1. Amidmonomer, dargestellt durch 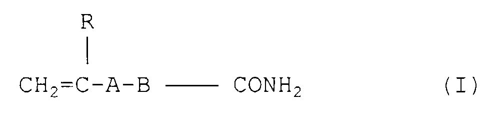 worin R ein Wasserstoffatom oder eine Alkylgruppe mit 1 bis 4 Kohlenstoffatomen und A einen Verbindungsteil, der durch 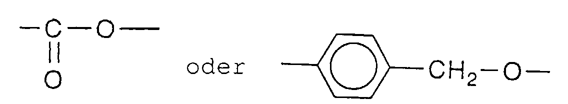 dargestellt wird, bedeuten,
B bedeutet einen Verbindungsteil, der durch 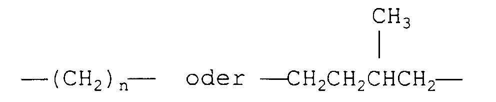 gezeigt wird, wobei n eine ganze Zahl von 3 bis 10 bedeutet.

2. Amidmonomer gemäß Anspruch 1, dargestellt durch 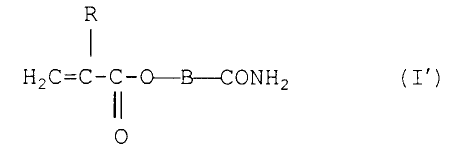 (worin R und B die obigen Bedeutungen aufweisen.)

3. Amidmonomer gemäß Anspruch 2, welches durch Reaktion einer Verbindung, die durch 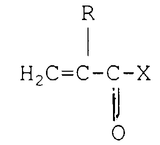 (worin X ein Chloratom, eine Hydroxylgruppe oder eine Alkoxygruppe mit bis zu 8 Kohlenstoffatomen bedeuten und R die gleiche Bedeutung wie oben aufweist, dargestellt wird,
mit einer Verbindung, die durch
J―B―CONH₂
(worin J ein Chloratom oder eine Hydroxylgruppe und B die obigen Bedeutungen aufweist) dargestellt wird, erhalten wird.

4. Amidmonomer gemäß Anspruch 3, worin die Kombination von X und J wie folgt ist:
X ist ein Chloratom, J ist eine Hydroxylgruppe;
X ist eine Hydroxylgruppe, J ist ein Chloratom oder eine Hydroxylgruppe; und
X ist eine Alkoxygruppe, J ist eine Hydroxylgruppe.

5. Amidmonomer gemäß Anspruch 1, dargestellt durch 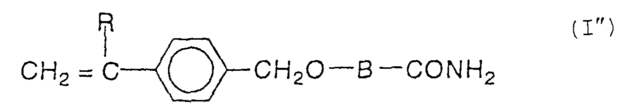 (worin R und B die obigen Bedeutungen aufweisen.)

6. Amidmonomer gemäß Anspruch 5, welches durch Reaktion einer Verbindung, die durch 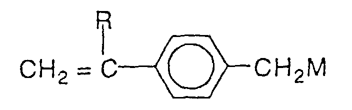 (worin R die obigen Bedeutungen aufweist und M ein Chloratom oder ein Bromatom ist), dargestellt wird,
mit einer Verbindung, die durch
HO―B―CONH₂
(worin B die obigen Bedeutungen aufweist) dargestellt wird, hergestellt wird.

7. Ein Polymer, hergestellt aus dem Monomer gemäß Anspruch 1.

8. Ein Polymer gemäß Anspruch 7, dargestellt durch die sich wiederholenden Einheiten: 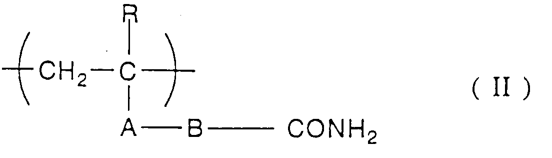 (worin R, A und B die obigen Bedeutungen aufweisen) 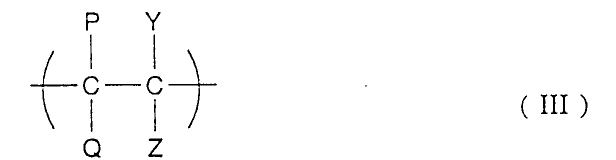 (worin P, Q, Y und Z unabhängig voneinander ein Wasserstoffatom; eine Alkylgruppe oder halogeniertes Alkyl mit 1 bis 6 Kohlenstoffatomen; ein Alken mit 1 bis 200 Kohlenstoffatomen; ein Aryl oder eine halogenierte Arylgruppe mit nicht mehr als 10 Kohlenstoffatomen; ein Aralkyl oder halogeniertes Aralkyl mit nicht mehr als 10 Kohlenstoffatomen; eine Cyangruppe; eine Mono- oder Dialkylaminogruppe mit 1 bis 6 Kohlenstoffatomen; eine Cyanaminoalkylgruppe mit 1 bis 6 Kohlenstoffatomen; eine Alkylcarbonyloxygruppe mit 1 bis 6 Kohlenstoffatomen; eine Arylcarbonyloxygruppe mit nicht mehr als 10 Kohlenstoffatomen; eine (Ph)ₖ-(CH₂)₁-CORₐ-Gruppe (worin Ph eine Phenylengruppe ist, Rₐ Hydroxyl, eine Alkoxygruppe mit 1 bis 10 Kohlenstoffatomen, eine Aryloxygruppe mit nicht mehr als 10 Kohlenstoffatomen, eine Aralkyloxygruppe mit nicht mehr als 10 Kohlenstoffatomen, eine Hydroxyalkoxygruppe mit 1 bis 6 Kohlenstoffatomen, Amino oder eine Mono- oder Dialkylaminogruppe mit 1 bis 6 Kohlenstoffatomen ist, k 0 oder 1 ist und 1 eine ganze Zahl von 0 bis 6 ist); oder eine (Ph)ₒ-(CH₂)ₚ-SO₂R_{b}-Gruppe (worin Ph das gleiche ist, wie es oben erwähnt wurde, R_{b} eine Hydroxylgruppe, eine Alkoxygruppe mit 1 bis 10 Kohlenstoffatomen oder eine Aralkyloxygruppe mit nicht mehr als 10 Kohlenstoffatomen ist, o 0 oder 1 ist, p eine ganze Zahl von 0 bis 6 ist); bedeuten),
worin die Anzahl der Einheiten (II) "s" ist, (s ist eine positive ganze Zahl) und die Anzahl der Einheiten (III) "t" ist (t ist 0 oder eine positive ganze Zahl) und die Amidgruppe in der Einheit (II) in einer Menge von 0,1 bis 28 Gew.% enthalten ist und das Polymer ein Molekulargewicht-Zahlenmittel von 500 bis 100.000 aufweist.

9. Polymer gemäß Anspruch 8, welches durch Polymerisieren des Amidmonomeren (I), dargestellt durch: 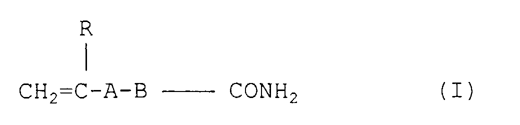 (worin R, A und B die gleichen Bedeutungen wie oben besitzen), und gegebenenfalls des Amidmonomeren (IV), dargestellt durch 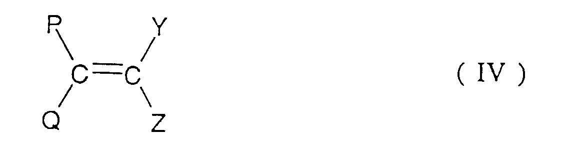 (worin P, Q, Y und Z die gleichen Bedeutungen wie oben besitzen), hergestellt wird.

## Revendications

1. Monomère amide représenté par la formule : 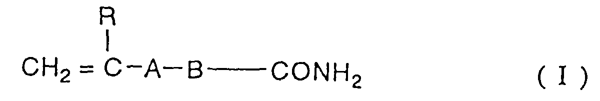 dans laquelle R représente un atome d'hydrogène ou un groupe alkyle ayant de 1 à 4 atomes de carbone et A signifie un membre de liaison représenté par 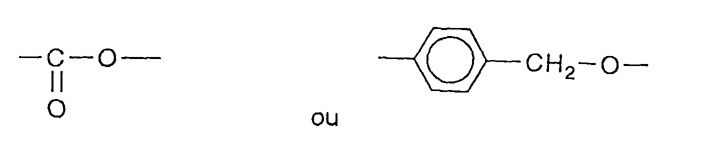 B représente un membre de liaison représenté par 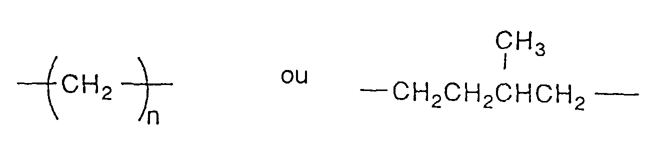 n représentant un nombre entier de 3 à 10.

2. Monomère amide selon la revendication 1 représenté par la formule : 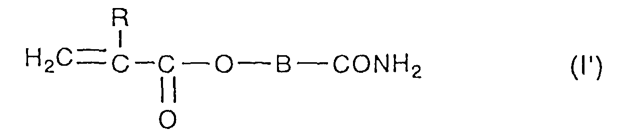 (dans laquelle R et B sont tels que définis ci-dessus).

3. Monomère amide selon la revendication 2 qui est obtenu en faisant réagir un composé représenté par la formule : 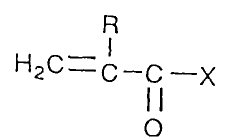 (dans laquelle X représente un atome de chlore, un groupe hydroxyle ou un groupe alkoxy ayant jusqu'à 8 atomes de carbone et R est tel que défini indiqué ci-dessus),
avec un composé représenté par la formule :
J―B―CONH₂
(dans laquelle J représente un atome de chlore ou un groupe hydroxyle et B est tel que défini ci-dessus).

4. Monomère amide selon la revendication 3 dans lequel la combinaison de X et J est la suivante :
X est un atome de chlore, J est un groupe hydroxyle ;
X est un groupe hydroxyle, J est un atome de chlore ou un groupe hydroxyle ; et
X est un groupe alkoxy, J est un groupe hydroxyle.

5. Monomère amide selon la revendication 1 représenté par la formule : 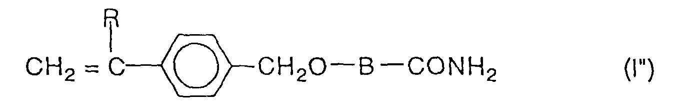 (dans laquelle R et B sont tels que définis ci-dessus).

6. Monomère amide selon la revendication 5 qui est obtenu en faisant réagir un composé représenté par la formule : 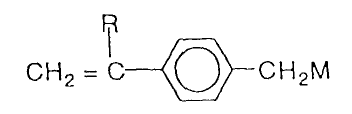 (dans laquelle R est tel que défini ci-dessus, M est un atome de chlore ou un atome de brome),
avec un composé représenté par la formule :
HO―B―CONH₂
(dans laquelle B est tel que défini ci-dessus).

7. Polymère préparé à partir d'un monomère selon la revendication 1.

8. Polymère selon la revendication 7 qui est représenté par les unités répétitives : 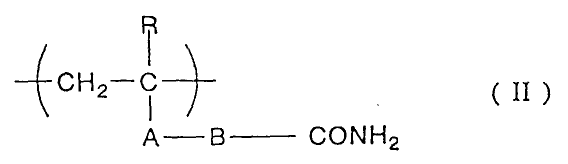 (R, A et B étant tels que définis ci-dessus), 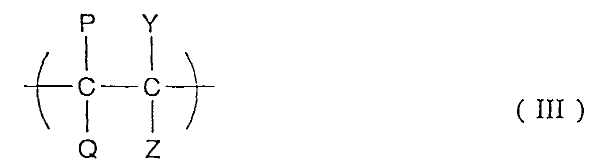 (P, Q, Y et Z représentent indépendamment un atome d'hydrogène, un groupe alkyle ou un alkyle halogéné ayant de 1 à 6 atomes de carbone ; un alcène ayant de 1 à 200 atomes de carbone ; un groupe aryle ou halogèno-aryle n'ayant pas plus de 10 atomes de carbone, un groupe aralkyle ou halogèno-aralkyle n'ayant pas plus 10 atomes de carbone ; un groupe cyano ; un groupe mono ou di-alkylamino ayant de 1 à 6 atomes de carbone, un groupe cyanaminoalkyle ayant de 1 à 6 atomes de carbone ; un groupe alkyle carbonyloxy ayant de 1 à 6 atomes de carbone ; un groupe aryl carbonyloxy n'ayant pas plus de 10 atomes de carbone ; un groupe (Ph)ₖ-(CH₂)ₗ-CORₐ (dans lequel Ph est un groupe phénylène, Rₐ est un hydroxyle, un groupe alkoxy ayant de 1 à 10 atomes de carbone, un groupe aryloxy n'ayant pas plus de 10 atomes de carbone, un groupe aralkyloxy n'ayant pas plus de 10 atomes de carbone, un groupe hydroxyalkoxy ayant de 1 à 6 atomes de carbone, un groupe amino ou mono ou di-alkylamino ayant de 1 à 6 atomes de carbone, k est 0 ou 1, l est un entier de 0 à 6) ; ou un groupe (Ph)_{O}-(CH₂)ₚ-SO₂R_{b} (dans lequel Ph est tel que défini ci-dessus, R est un groupe hydroxyle, un groupe alkoxy ayant de 1 à 10 atomes de carbone ou un groupe aralkyloxy n'ayant pas plus de 10 atomes de carbone, o est 0 ou 1, p est un entier de 0 à 6),
dans lequel le nombre d'unité (II) est "s" (s est un entier positif) et le nombre d'unité (III) est "t" (t est 0 ou un entier positif) et le groupe amide dans l'unité (Il) est présent en une proportion de 0,1 à 28% en poids et le polymère a un poids moléculaire moyen en nombre de 500 à 100 000.

9. Polymère selon la revendication 8 obtenu par polymérisation du monomère amide (I) représenté par la formule : 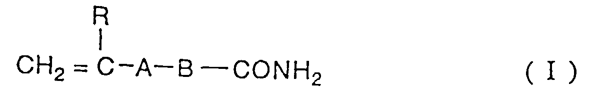 (dans laquelle R, A et B sont tels que définis ci-dessus), et éventuellement le monomère (IV) représenté par la formule : 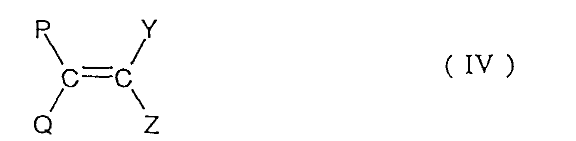 (dans laquelle P, Q, Y et Z sont tels que définis ci-dessus).
